## Europäisches Patentamt

## European Patent Office

## Office européen des brevets

(11) Publication number: **0 139 143**
**B1**

(12) **EUROPEAN PATENT SPECIFICATION**

(45) Date of publication of patent specification: **07.01.88**

(51) Int. Cl.⁴: **C 12 Q 1/70,** C 12 M 1/34, C 12 N 7/00

(21) Application number: **84109775.1**

(22) Date of filing: **16.08.84**

(54) **Process and apparatus for measuring the parameters characterizing the microbiological interactions between phages and bacteria.**

(30) Priority: **23.08.83 HU 295283**

(43) Date of publication of application:
**02.05.85 Bulletin 85/18**

(45) Publication of the grant of the patent:
**07.01.88 Bulletin 88/01**

(84) Designated Contracting States:
**AT CH DE FR GB IT LI**

(56) References cited:

**ZEITSCHRIFT FÜR ALLGEMEINE MIKROBIOLOGIE, vol. 19, 1979, Berlin S. GASPAR, GY. RONTO and G. MÜLLER "Determination of the biological parameters of bacterium-phage complexes" pages 163-169**

(73) Proprietor: **RADELKIS Elektrokémiai Müszergyárto Ipari Szövetkezet Laborc utca 1 H-1300 Budapest (HU)**

(73) Proprietor: **Magyar Tudományos Akadémia Természettudományi Kutatolaboratoriumai Biofizikai Kutatolaboratoriuma Puskin utca 9 H-1444 Budapest (HU)**

(72) Inventor: **Derka, István 20, Vöröstorony utca H-1023 Budapest (HU)**
Inventor: **Gáspár, Sándor 85, Pesti út H-1173 Budapest (HU)**
Inventor: **Módos, Károly 39, Pesti út H-1173 Budapest (HU)**
Inventor: **Ronto, Györgyi, Dr. M.D. 150 Öv utca H-1141 Budapest (HU)**
Inventor: **Tarjan, Imre, Dr. 145 Fehérvári út H-1119 Budapest (HU)**
Inventor: **Erdélyi, János 129 Vöröshadsereg ut H-1021 Budapest (HU)**

**0 139 143**

(72) Inventor: **Müller, Henrik**
**54 Péterhegyi köz**
**H-1112 Budapest (HU)**

(74) Representative: **Hansen, Bernd, Dr.rer.nat. et al**
**Hoffmann, Eitle & Partner Patentanwälte**
**Arabellastrasse 4**
**D-8000 München 81 (DE)**

## Description

The invention relates to a process and an apparatus for the realization of the process according to the invention with the purpose to determine during the approximate cycle time of phage development the parameters of phage development cycle having been defined in the course of the interactions between virulent phages and carrier-bacteria (e.g. $\mu$, the velocity constant of adsorption, $T$ the time of phage development, and standard deviation thereof $\sigma$, phage-yield $C$ to be expected, multiplicity index of infection $m_i$), i.e. to measure biological changes realized in the change of parameters caused by the physical and/or chemical effects affecting the phages or bacteria or the complexes thereof.

The process and the equipment according to the invention can be advantageously applied e.g. in the pharmaceutical and chemical industry, in agriculture, in the field of ecology and everywhere, where there is a demand for the quantitative evaluation of the biological (molecular biological) effects of physical and/or chemical agents.

About phages, as biological objects and macromolecules, much is known in respect of biological function and structure. In contrast, application of the phages for practical (biotechnological) purposes—as a possibility—has not been extensively utilized up to now. Taking the increased demand for quantitative biological measurements and tests into consideration, it seems to be most probable that application of phage-bacteria complexes—due to their well definable and measurable, quantitatively characterizable interactions—will come into the limelight.

Quantities characterizing phage development cycle and determination thereof were defined and summarized in the classical work of Adams (Adams, J. M. Bacteriophages, Intersci. Publ. New York). The methods described therein have been used ever since, however, with more or less modification. Grossi et al. (Grossi G. F. Cesarini, G. and Liello, F. 1977. Development of phage populations in a bacterial culture: A mathematical model. Z. Naturforsch. *32* 844—849) elaborated the method for the determination of the parameters characterizing the cycle of phase development, being more accurate, than the previous one. The disadvantage of this work, however, lies in that their method is based on the experimental results of other authors, so the lack of their own experimental background inhibited further development of the model as well as propagation of application. Gáspár et al. (S. Gáspár, Gy. Rontó and G. Müller 1979. Determination of the biological parameters of bacterium-phage complexes. Zeitschrift für Allg. Mikrobiol. *19* 163—169) performed experiments directed to measuring the parameters characterizing the phage development cycle and mathematical models co-ordinated with experimental conditions were prepared.

In such a manner parameter-values having been defined by said process were reliable and accurate but at the same time the preparatory work and performance of measurements needed were utmost complicated and the evaluation of the results was time consuming.

Concentration measurements based on the interaction between bacterium and phage, the determination of the so-called plague forming activity, has been frequently used for detecting certain physical or chemical damaging effects.

Thus e.g. Rontó et. al. examined the damaging effect of UV radiation and radiomimetics on phages, wherein the experimental data given in the reporting publication, i.e. measurements, represent the result of minimally several months of research activity (Rontó Gy., Sarkadi, K. und Tarján, I.: 1967. Zur Analyse der UV-Dosiswirkungskurven der T7-Phagen, Strahlenter. *134* 151—157; Karczag, A., Rontó, Gy. and Tarján, I.: 1972, Study of the UV-effect on MS2 phages. Acta Biochem. Biophys. Hung 7 173—177; Rontó, Gy., Smotryaeva, M, Kruglyakova, K., Emmanuel, N., and Tarján, I.: 1975. Inactivation of bacteriophages T7 and $\phi$X 174 by radiomimetics. Acta Biochem. Biophys. Hung. *10* 115—122).

The Hungarian Patent No. 183101, corresponding to EP—A—115547 relating to a measuring (qualifying) process based on the lethal mutation of the phage T7 of the mutagenic effect of chemical substances, led back the measuring process to the determination of plague forming activity in a traditional manner.

Parameters characterizing the phage development cycle can be used as reliable measurement information for characterizing different processes, if good reproducibility of measurement, reliability and quick automatic performance can be ensured. For the sake of reproducibility with certain part-processes time constancy of the bacterium-suspension, the biological state being suitable for measurement, as well as concentration needed for measurement must be ensured. A further task lies in the elimination of the statistical errors of manual processes according to original descriptions (plague counting titration). Simultaneously, in order to be able to eliminate subjective errors resulting from plague counting it seems to be expedient to search macroscopic characteristics, which are in an unambiguous correlation with the number of bacteria and can be measured in an automatized manner. Furthermore, it seems to be expedient to measure the parameters—compared to known methods—more accurately and within the possible shortest time (if possible, during the burst time).

Time constancy of the bacterium suspension and concentration needed for measuring can be ensured by a continuously operated fermentor. A further task of the fermentor is to maintain the proper biological state of the bacteria to be able to separate phage development cycles following each other in the interest of simple evaluation. In order to be able to eliminate statistical errors infection with a high multiplicity was used which led within a shorter time (during one burst) to changes which could be detected also by physical/chemical characteristic. In this state namely the phages dissolve the bacteria (lysis). The process can be measured by detecting the number of bacteria, or any light-scattering, light absorption, ion-environment etc. being in an unambiguous connection with the number of bacteria. In the case of an infection of high multiplicity (about 1), the ratio signal/noise will be optimal which enables accurate determination of multiplicity and by the derivation of the measured signals measuring of the parameters T and σ. For the quick determination of the expected phage-yield bacteria used to be infected with a multiplicity of $m_i > 3$, in this case at the end of the phage development cycle a complete lysis takes place. On the lysate thus obtained—containing also the newly developed phages—measuring of the multiplicity $m_i$ can be repeated by the known dilution. The product of the multiplicity having been determined in such a manner and of the dilution applied give the expected phage-yield $C$. Accordingly, independently of the bacterium concentration the measurement determines the expected numbers of phages arising from one bacterium during the period of two bursts.

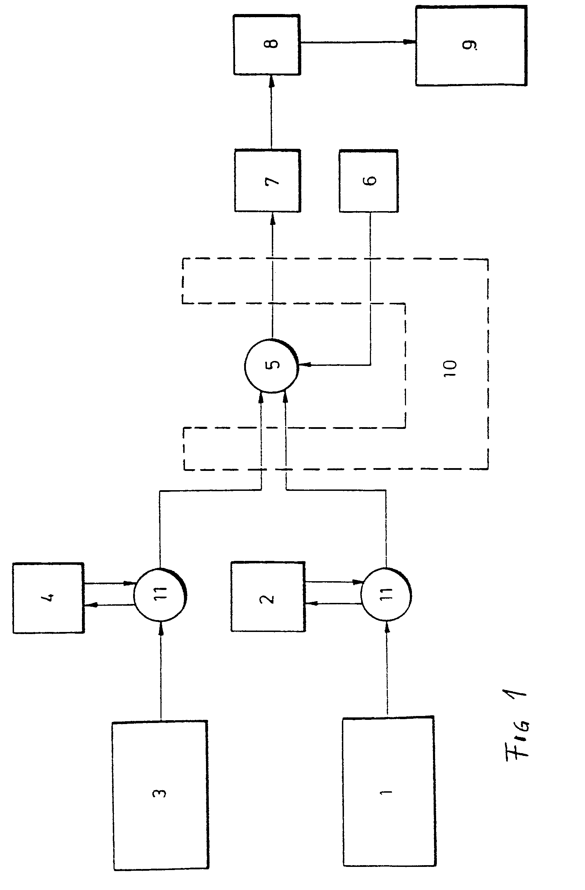

The advantage of the specified process lies in that for its realization the equipment can be organized unit per unit in a separated form, principal layout thereof can be seen in the drawing. The equipment for performing the process according to the invention consists of the independent fermentor 1, wherefrom bacteria in the needed quantity arrive through the charger 2 into the biological reactor 5; from the recipient 3 storing the phages the suitable phage quantity was already led into said reactor via the diluting unit 4. Gas-charger 6 ensures mixing, optional oxygen supply or closing off from oxygen. At the beginning of the measuring cycle a part of the sample arrives by the aid of the liquid forwarding unit 8 into the measuring unit 7; if it is possible, in order to ensure continuous measuring, unit 7 should be designed with a flow-type character or so, that it could be used as a reaction space (introduction of gas, thermostating). At the end of the completed phage development cycle the sample returns to the measuring unit 7, and after the measurement it arrives to the collector 9.

The process and the equipment according to the invention—as specified above—is for measuring the characterizing parameters of the phage-bacterium complex, i.e. the measurement process can be repeated, if the critical part of the system—the biological reaction space—is flushed between two measuring cycles. Distilled water is well suitable for this purpose, as the majority of the active phages disturbing the following measuring cycle is irreversibly inactivated in the ion-free water.

Both the process and the equipment are well suitable for measuring biological consequences of certain (physical and/or chemical) effects in the following manner:

I. Inactivation test

By dialysis the purified phages can be dissolved to give an optional solution and can be stored therein practically for an unlimited time. In the solution the phages behave as unliving objects as long as they do not contact with their host cells (bacteria). The phages having the properties of living matter but not showing the symptoms of life in the solution, can be subjected to well defined physical and/or chemical effects and in accordance with the process according to the invention we can test all the biological consequences of the structural change occurring under the influence of treatment.

The recipient 3 storing the phages is well suitable for treating the phages (e.g. at a given temperature, incubation with chemicals of given concentration and for a given period), while the effect (resulting frequently in a reduced activity, i.e., in the change of multiplicity) can be measured by the apparatus.

2. Cell-virus test

In the case where an active phage arrives at a bacterium cell instead of the normal, information relating to the function of bacteria information needed for producing phages will be obtained. As already mentioned, parameters of the phage development cycle depend considerably on the biological state of the bacteria. Thus e.g. bacteria having been subjected to different treatments in the fermentor can be tested on the basis of the parameters of phage-production. In case of the proper selection of the bacterium-phage system, the part-process of other cell-virus interactions can be also modelled.

Intervention into the system is taking place through the fermentor 1 of the apparatus, in the measurement where the phages are untreated.

3. Phage-programme test

In the case where in the course of the phage-bacterium interaction the μ adsorption rate constant is high, phage development process in the bacteria contained in the sample-space is taking place approximately synchronized. The influences affecting the system are contacting the given phases of the phage-producing programme in the phage development cycle in the optional moment, accordingly, change in parameters is giving information, which is characteristic for the influence effecting the complex. In such a manner photosensitizers, chemical and/or physical effects inhibiting or promoting metabolism—which can be activated in the optional moment of the phage development cycle can be maintained in the activated state for the optional period—can be tested in a quite new manner.

With the apparatus according to the invention the effect of the photo-sensitizer can be advantageously measured, if the measuring system works on an optical principle and the activating light is perpendicular to the measuring light path.

In the course of a composite measuring process—i.e. the system contains at least two of fermentors 1 and/or recipients 3 storing the phages, between the fermentors 1 in itselves there is an OP- connection: from any of the fermentors 1 or the recipients 3 storing the phages the materials can get through an AND-connection into one of the biological reaction spaces 5, which are then placed necessarily in the automatic sample changes 10. It goes without saying that the number of the chargers 2 and diluting units 4 must be in compliance with the number of the fermentors 1 and recipients 3. In the proper moment the sample arrives serially from the sample charger 10 into the measuring unit 7 and therefore into the collector 9. As it becomes obvious, the composite system can be advantageously automatized and applied, while automatics may perform evaluation and recording of the measurement.

Example

The process and the apparatus according to the invention—which serve for measuring the interaction between phages and bacteria (parameters characterizing biological) interaction)—will de detailed by illustrating the measurement of the mutagenic effect, as described in the Hungarian Patent 183101 from which all the advantages of our method and apparatus, such as speed, simplicity, reproducibility and possibility of automatization become obvious.

The task to be solved: following the remnant activity of T7 phages incubated with a chemical in dependence of time. Expediently, the apparatus can be realized in the following composition:

A fermentor, functioning as a chemostat, that means, that the ratio of the volume of a culture vessel provided with an overflow and the velocity of the fed culture medium defines the growth rate of the bacteria, which is constant in time.

A bacterium charger, which is delivering the bacterium suspension of a given volume into the biological reaction spaces.

Two recipients for storing the phages, into which the phages having been treated with a reagent or those serving for control are arranged.

Two parallel functioning diluting units, which are forwarding the diluted treated phages and control-phages, respectively into the biological reaction spaces.

Obviously, the reaction spaces are arranged in an automatic sample charger and are thermostated to a temperature optimal for the host bacteria.

Optionally, the gas feeder can lead $O_2$ gas into any reaction space. The measuring unit is operating on optical basis, it is giving an optical signal proportional to the number of non-lyzed bacteria. For the performance of the measuring process the apparatus is provided with a cuvette with the flow character and thermostated to the proper temperature. A peristaltic pump delivers the samples from the reactor via a cuvette into the collector.

From the magnitude of the optical signals measured immediately after infecting the bacteria and at the end of the cycle multiplicity of phages ready to infect can be easily deducted. In the course of the determination it should be considered that partly the phages are adsorbed on the bacteria according to the Poisson distribution, partly two distortioning factors are to be considered, in so far as, the bacteria fall apart to not-completely transparent parts in course of lysis, furthermore during the lysis-period uninfected bacteria are further growing.

In the fermentor the B. coli B/r host bacteria are continuously incubated, e.g. division time amounts to 1,6 h with an M9 minial nutrient enriched with 1,5 g/l glucose and 1,5 g/l casaminoacid.

The proper solution of the chemical to be measured and the control solution (containing merely a solvent) are mixed with the needed quantity of purified bacteriophage (e.g. in a concentration of $10^{11}$/ml). Thereafter the solutions are led into the suitable recipients storing the phages. At proper moments of measuring the diluting units allow the flow of identically diluted samples from both of the treated and control samples, respectively, into the reaction spaces (e.g. 1 ml each per sample space in a tenfold dilution). As a diluting solution the nutrient is used.

Thereafter the bacterium charger feeding at equal intervals (e.g. every minute) host-bacteria into the reaction spaces (so e.g. 3 ml each into the reaction spaces of a six-number sample changer), meanwhile oxygen is bubbled through the substance to obtain proper mixing.

The samples in the reaction spaces are freshened by a throughflow of $C_2$ from time to time (e.g. at least once in every minute for a period of minimum 5 seconds), just up to the beginning of the lysis (e.g. into each single sample space from feeding the bacteria up to the 14th minute).

From the reaction space containing a selected dilution of the treated phages, a part of the sample is transferred into the optical measuring device by the aid of a peristaltic pump. By the continuous measurement and derivation of the optical signal we are monitoring, whether latency time and its standard deviation (e.g. 18 minutes and 3 minutes, resp.) comply with prescriptions (biological control). Where the biological control does not meet the requirements, the diluted chemical may influence metabolism of the host bacteria, which excludes evaluation of the measurement.

In case of suitable values of biological control, after having finished lysis (e.g. in the 26th minute after bacteria feeding) the samples are suctioned one after the other—in the rhythm of bacteria feeding—through the measuring cuvette, and the magnitude of the optical signals is registered.

After finishing the measurement and rinsing the reaction spaces with distilled water the process can be repeated from the next measurement (e.g in every forty minutes).

At the end of a measurement series on basis of the magnitude of the optical signals having been defined in points of time belonging to the single samplings the change in the concentration of the treated sample related to the control sample can be calculated, i.e. we may obtain the inactivation kinetics of the chemical used.

Considering the necessity of continuous control and strict time correlation of the aforementioned processes, it seems to be expedient to carry out measuring with an automatic control and data processing (microprocessored system).

The process according to the invention helps to solve actual biological problems, as e.g. quick recognition of the mutagenic effect of chemicals i.e. quantitative evaluation thereof, as well as promotion of research of substances effecting viri and bacteria i.e. physical effects exerted thereon.

Quickly obtainable and reliable data of the (automatized) apparatus having been assembled according to the given task, enables application for biotechnological purposes and useful application of dynamically developing biological science.

## Claims

1. A process for measuring parameters of the phage development cycle occurring under the effect of interaction between bacteria and phages, using detection methods known per se, characterized by

(a) mixing phages and host-bacteria, and then measuring continuously physical and/or chemical characteristics as a functional correlation of the number of bacteria during a single phage development cycle,

(b) determining the index of infection multiplicity $[m_i]$ from the values of the signals measured at the beginning and at the end of the phage development cycle,

(c) determining by means of the zero-point of the second derivative of the value according to the period of the first phage development cycle the latence time (T),

(d) determining from the distance of the two zero-points of the third derivative of the above value the standard deviation of latence time ($\sigma$), and

(e) diluting the total lysate obtained in the end of the phage development cycle, mixing the diluted lysate with the same host-bacteria concentration, measuring again the multiplicity in the new cycle and evaluating the value of the phage yield [C] in a manner known per se as the product of dilution and multiplicity.

2. Apparatus for measuring the microbiological interactions between bacteria and phages containing bacteria source(s), space(s) for storing the phages, reaction space(s) for establishing the interaction and measuring device(s), characterized in that the bacterium producing fermentor (1) is connected to the biological reaction space (5) through a charger (2), space (3) for storing the phages and diluting unit (4), according to necessity gas can be fed through a feeder (6) into said reaction space which is connected to the collector (9) via the measuring unit (7) and the unit (8) forwarding the liquid.

3. Process as claimed in claim 1, characterized in that the change in the parameters of the phage development cycle occurring as a result of a physical and/or chemical effect exerted on the phages (inactivation test) is determined.

4. Process according to claim 1, characterized in that the change of the parameters of the phage development cycle occurring as a result of the physical and/or chemical effect exerted on the bacterium-phage complex (phage-programme test) is determined.

5. Process as claimed in claim 1, characterized in that the change of the parameters of the phage development cycle occurring as a result of physical and/or chemical effect exerted on bacteria (cells-virus test) is determined.

6. Apparatus as claimed in claim 2, characterized in that the fermentor is operated as a chemostat.

7. Apparatus as claimed in claim 2 and 6 characterized in that at least one measuring unit (7) is operated on optical principles.

8. Apparatus as claimed in claim 2 and 6, characterized in that at least one measuring unit (7) is operated on electrochemical principles.

9. Apparatus as claimed in claim 2 and 6, characterized in that at least one measuring unit (7) is a cell-counter.

10. Apparatus as claimed in claims 2 and 6 to 9, characterized in that for simultaneous related, resp. parallel measurings two or more fermentors (1) phage-chargers (2), recipients (3) for storing, diluting units (4) and reaction spaces (5), automatic sample changers (10) performing the connection between said components are used.

11. Apparatus as claimed in any of claims 2 and 6 to 10, characterized in that the surfaces contacting biological substances are made of a hydrophobic material.

12. Apparatus as claimed in claims 2 and 6 to 11, characterized in that the valves regulating the flow consist of pipes in a closed system and are operated by pipe-break.

## Patentansprüche

1. Verfahren zur Messung der Parameter des Phagen-Entwicklungszyklus als Effekt der Wechselwirkung zwischen Bakterien und Phagen mit Hilfe von an sich bekannter Nachweismethoden, gekennzeichnet durch

(a) Vermischen von Phagen und Wirt-Bakterien und anschliessende kontinuierliche Messung physikalischer und/oder chemischer Charakteristiken als funktionnelle Korrelation der Bakterienzahl während eines einzigen Phagen-Entwicklungszyklus,

(b) Bestimmen des Index der Infektionsmultiplizität $[m_i]$ aus den Werten der zu Beginn und am Ende des Phagen-Entwicklungszyklus gemessenen Signale,

(c) Bestimmen der Latentszeit (T) mit Hilfe des Null-Punktes der zweiten Ableitung des Wertes entsprechend der Zeitdauer des ersten Phagen-Entwicklungszyklus,

(d) Bestimmen der Standardabweichung der Latenzzeit (sigma) aus dem Abstand der zwei Nullpunkte der dritten Ableitung des vorstehend Wertes, und

(e) Verdünnen des am Ende des Phagen-Entwicklungs-zyklus erhaltenen gesamten Lysats, Vermischen des verdünnten Lysats mit der gleichen Konzentration an Wirt-Bakterien, erneutes Messen der Multiplizität im neuen Zyklus und Bestimmen des Wertes der Phagen-Ausbeute (C) in an sich bekannter Weise als Produkt der Verdünnung und Multiplizität.

2. Vorrichtung zur Messung mikrobiologischer Wechselwirkungen zwischen Bakterien und Phagen, welche umfasst: Bakterienquelle(n), Raum bzw. Räume zum Aufbewahren der Phagen, Reaktionsraum bzw. -räume, in welchen die Wechselwirkung stattfindet, und Messvorrichtung(en), dadurch gekennzeichnet, dass der Bakterien-produzierende Fermenter (1) mit dem biologischen Reaktionsraum (5) über eine Beschickungseinrichtung (2) in Verbindung steht, Raum (3) zum Aufbewahren der Phagen und Verdünnungseinheit (4), wobei erforderlichenfalls Gas über die Zuführleitung (6) in den genannten Reaktionsraum, der mit dem Sammelbehälter (9) über die Messeinheit (7) und die Einheit (8), welche die Flüssigkeit weiterleitet, verbunden ist, zugeführt werden kann.

3. Verfahren nach Anspruch 1, dadurch gekennzeichnet, dass die Veränderung der Parameter des Phagen-Entwicklungszyklus, die sich aufgrund eines physikalischen und/oder chemischen Effektes, der auf die Phagen ausgeübt wird, ergibt (Inaktivierungstest) bestimmt wird.

4. Verfahren nach Anspruch 1, dadurch gekennzeichnet, dass die Veränderung der Parameter des Phagen-Entwicklungszyklus, die sich aufgrund eines physikalischen und/oder chemischen Effektes, der auf den Bakterien-Phagen-Komplex ausgeübt wird, ergibt (Phagen-Programm-Test), bestimmt wird.

5. Verfahren nach Anspruch 1, dadurch gekennzeichnet, dass die Veränderung der Parameter des Phagen-Entwicklungszyklus, die sich aufgrund eines physikalischen und/oder chemischen Effektes, der auf die Baktieren ausgeübt wird, ergibt (Zell-Virus-Test), bestimmt wird.

6. Vorrichtung nach Anspruch 2, dadurch gekennzeichnet, dass der Fermenter als Chemostat geführt wird.

7. Vorrichtung nach Anspruch 2 und 6, dadurch gekennzeichnet, dass mindestens eine Messeinheit (7) ein optisches Messinstrument darstellt.

8. Vorrichtung nach Anspruch 2 und 6, dadurch gekennzeichnet, dass mindestens eine Messeinheit (7) eine elektrochemische Messeinheit darstellt.

9. Vorrichtung nach Anspruch 2 und 6, dadurch gekennzeichnet, dass mindestens eine Messeinheit (7) eine Zellenzählvorrichtung darstellt.

10. Vorrichtung nach Ansprüchen 2 und 6 bis 9, dadurch gekennzeichnet, dass für gleichzeitige Messungen bzw. Parallelmessungen zwei oder mehr Fermenter (1), Phagen-Beschickungseinrichtungen (2), Vorratsbehälter (3), Verdünnungseinheiten (4) und Reaktionsräume (5), automatische Probenwechsler (10), die die Verbindung zwischen den genannten Komponenten herstellen, verwendet werden.

11. Vorrichtung nach einem der mehreren der Ansprüche 2 und 6 bis 10, dadurch gekennzeichnet, dass die· Oberflächen, die in Kontakt mit biologischen Substanzen stehen, aus hydrophobem Material sind.

12. Vorrichtung nach Ansprüchen 2 und 6 bis 11, dadurch gekennzeichnet, dass die Ventile, welche den Zu- und Abfluss regulieren, aus Röhren in einem geschlossenen System bestehen und mittels einer Rohrunterbrechung betätigt werden.

**Revendications**

1. Procédé de mesure de paramètres du cycle de développement de phages se produisant sous l'effet d'une interaction entre bactéries et phages en utilisant des méthodes de détection connues en soi, caractérisé en ce que:

a. on mélange des phages et des bactéries hôtesses et l'on mesure ensuite, en continu, des caractéristiques physiques et/ou chimiques, à titre de corrélation fonctionnelle du nombre de bactéries pendant un cycle unique de développement d'un phage;

b. on détermine l'indice de multiplicité d'infection $(m_i)$ ä partir des valeurs des signaux mesurées au début et à la fin du cycle de développement du phage;

c. on détermine, au moyen du point zéro de la dérivée seconde de la valeur selon la période du premier cycle de développement du phage, le temps de latence (T);

d. on détermine, à partir de la distance de deux points zéro de la dérivée troisième de la valeur ci-dessus, la déviation standard du temps de latence (σ); et

e. on dilue le lysat total obtenu à la fin du cycle de développement du phage, on mélange le lysat dilué à la même concentration de bactéries-hôtesse, on mesure à nouveau la multiplicité dans le nouveau cycle et l'on évalue la valeur du rendement (C) en phage d'une façon connue en soi comme étant le produit de la dilution et de la multiplicité.

2. Dispositif de mesure des interactions microbiologiques entre bactéries et phages comprenant des source(s) de bactéries, des espace(s) de stockage des phages, des espace(s) de réaction pour établir l'interaction et des dispositif(s) de mesure, caractérisé en ce que le bac de fermentation (9) produisant les bactéries est relié à l'espace (5) de réaction biologique par un chargeur (2), un espace (3) de stockage des phages et une unité de dilution (4), du gaz pouvant être introduit en cas de nécessité par un dispositif d'alimentation (6) dans cet espace de réaction relié au collecteur (9) par l'unité de mesure (7) et l'unité (8) d'acheminement du liquide.

3. Procédé selon la revendication 1 caractérisé en ce que l'on détermine le changement des paramètres du cycle de développement du phage survenant à titre de résultat d'un effet physique ou chimique exercé sur les phages (test d'inactivation).

4. Procédé selon la revendication 1, caractérisé en ce que l'on détermine le changement des paramètres du cycle de développement du phage apparaissant comme un résultat de l'effet chimique et/ou physique exercé sur le complexe bactérie-phage (test phage-programme).

5. Procédé selon la revendication 1, caractérisé en ce que l'on détermine le changement des paramètres du cycle de développement du phage apparaissant comme résultat de l'effet chimique et/ou physique exercé sur les bactéries (test cellule-virus).

6. Dispositif selon la revendication 2, caractérisé en ce que le bac de fermentation fonctionne comme un bac de culture de continu des bactéries.

7. Dispositif selon la revendication 2 et 6, caractérisé en ce qu'au moins l'une des unités de mesure (7) fonctionne sur des principes optiques.

8. Dispositif selon la revendication 2 et 6, caractérisé en ce qu'au moins l'une des unités de mesure (7) fonctionne sur des principes électrochimiques.

9. Dispositif selon les revendication 2 et 6, caractérisé en ce qu'au moins l'une des unités de mesure est un compteur de cellules.

10. Dispositif selon la revendication 2 et 6 à 9, caractérisé en ce que pour des mesures simultanées correspondantes ou parallèles, on utilise au moins deux bacs de fermentation (1), chargeurs de phages (2), récipients (3) de stockage, unités de dilution (4) et espaces de réaction (5), des chargeurs automatiques d'échantillon (10) réalisant la connexion entre lesdits composants.

11. Dispositif selon l'une quelconque des revendications 2 et 6 à 10, caractérisé en ce que les surfaces entrant en contact des substances biologiques sont en matériau hydrophobe.

12. Dispositif selon l'une quelconque des revendications 2 et 6 à 11, caractérisé en ce que les vannes de régulation des écoulements sont constituées de tuyaux en circuit fermè et fonctionnent par rupture de tuyaux.

FIG 1